# EUROPÄISCHE PATENTSCHRIFT

(11) **EP 0 975 568 B1**
(45) Veröffentlichungstag und Bekanntmachung des Hinweises auf die Patenterteilung: **21.11.2001**
(21) Anmeldenummer: 98901942.7
(22) Anmeldetag: 07.01.1998
(51) Int. Cl.: C07C 37/20, C07C 39/16

(54) **VERFAHREN ZUR HERSTELLUNG VON BIS-(4-HYDROXYARYL)-ALKANEN**
PROCESS FOR PREPARING BIS-(4-HYDROXYARYL-)-ALKANES
PROCEDE DE PREPARATION DE BIS-(4-HYDROXYARYL)-ALCANES

(30) Priorität: 16.01.1997 DE 19701278
(43) Veröffentlichungstag der Anmeldung: 02.02.2000
(73) Patentinhaber: BAYER AG, 51368 Leverkusen (DE)
(72) Erfinder: FENGLER, Gerd, D-47802 Krefeld (DE); BUYSCH, Hans-Josef, D-47809 Krefeld (DE); HEYDENREICH, Frieder, D-40593 Düsseldorf (DE); EEK, Rob, D-51061 Köln (DE); FENNHOFF, Gerhard, D-47877 Willich (DE)
(86) Internationale Anmeldenummer: EP9800034
(87) Internationale Veröffentlichungsnummer: WO9831651

(56) Entgegenhaltungen:
- EP-A- 0 754 666
- US-A- 4 391 997
- US-A- 4 400 555

## Beschreibung

Die vorliegende Erfindung betrifft ein Verfahren zur Herstellung von Bis-(4-hydroxyaryl)-alkanen durch heterogen katalysierte Umsetzung aromatischer Hydroxyverbindungen mit Ketonen in in Reihe geschalteten Reaktoren, die in Richtung fortschreitender Umsetzung mit steigender Temperatur betrieben werden, wobei die Gesamtmenge an Keton auf die einzelnen Reaktoren verteilt wird und zwar so, daß mit steigender Temperatur die Menge kleiner wird.

Es ist bekannt, in einem Prozeß zur Herstellung von Bis(4-hydroxyaryl)alkanen aus Phenolen und Ketonen die erforderliche Ketonmenge auf mehrere in Reihe geschaltete Reaktoren zu verteilen. Die US-PS 2 775 620 beschreibt ein mit Mineralsäure katalysiertes Verfahren in homogener flüssiger Phase, ein heterogen katalysiertes Verfahren mit einem sauren Ionenaustauscher in einem Festbettreaktor geht aus US-PS 4 400 555 hervor. Beide Schriften belegen, daß durch Aufteilung der Ketonmenge der Anteil an Nebenprodukten reduziert wird, wobei dieser Anteil bei HCl-Katalyse geringer ist als bei Katalyse mit Ionentauschern.

US-A 4 391 997 offenbart, dass bei der Herstellung von Bis(4-hydroxyaryl) alkanen in Richtung fortschreitender Umsetzung steigende Temperaturen vorteilhaft sind.

Die Katalyse der Reaktion mit HCl unterscheidet sich in chemischer und verfahrenstechnischer Hinsicht deutlich von der mit heterogenen Katalysatoren. In der Regel ist die HCl-Katalyse selektiver und kann außerdem bei niedrigeren Temperaturen durchgeführt werden, da eine Kristallisation in den Reaktoren toleriert werden kann oder sogar für eine höhere Selektivität von Vorteil ist. So wird in US-PS 2 775 620 auch konsequent unterhalb der Kristallisationstemperatur von 63°C gearbeitet und eine hohe Selektivität erzielt. Ganz anders in US-PS 4 400 555, wo die Temperatur bei 67°C gehalten wird, um eine Kristallisation und damit eine Verstopfung des (heterogenen) Katalysatorbettes zu vermeiden. In den Beispielen wird bei einem Acetonumsatz von 66 % gearbeitet. Die Erhöhung der Selektivität fällt deutlich niedriger aus als in der HCl-katalysierten Reaktion. Außerdem wird festgestellt, daß die Selektivität weiter steigt, wenn die größere Acetonmenge dem 2. Reaktor zugeführt wird.

Die nach US-PS 4 400 555 gefundene Selektivitätsverbesserung für Bis-(4-hydroxyaryl)-alkane ist noch unzureichend. Daher ist es wünschenswert, Verfahren zu entwickeln, bei denen unter den Bedingungen einer kontinuierlich betriebenen Anlage möglichst wenig an Isomeren und vor allem an Nebenprodukte gebildet werden.

Es wurde nun gefunden, daß man deutlich höhere Selektivitäten an Bis-(4-hydroxyaryl)-alkanen erhält, wenn man die Gesamtmenge an Keton so auf die Reaktoren verteilt, daß dem einzelnen Reaktor umso weniger Keton zudosiert wird, je höher seine Temperatur ist, die Belastung der Reaktoren möglichst groß ist und die Temperatur entlang der Reaktorkette zwar steigt, aber vergleichsweise möglichst niedrig gehalten wird.

Gegenstand der Erfindung ist daher ein Verfahren zur Herstellung von Bis-(4-hydroxyaryl)-alkanen durch heterogen katalysierte Umsetzung von aromatischen Hydroxyverbindungen mit Ketonen in mindestens zwei in Reihe geschalteten Reaktoren, die in Richtung fortschreitender Umsetzung bei steigenden Temperaturen betrieben werden, wobei die Gesamtmenge an Keton auf die einzelnen Reaktoren so aufgeteilt wird, daß der Anteil pro Reaktor umso kleiner ist, je höher die Temperatur des jeweiligen Reaktors ist.

Geeignete aromatische Hydroxyverbindungen für das erfindungsgemäße Verfahren sind in p-Position nicht substituiert und enthalten keine Substituenten zweiter Ordnung wie Cyano-, Carboxy- oder Nitrogruppen; genannt seien beispielsweise Phenol, o- und m-Kresol, 2,6-Dimethylphenol, o-tert.-Butylphenol, 2-Methyl-6-tert.-butylphenol, o-Cyclohexylphenol, o-Phenylphenol, o-Isopropylphenol, 2-Methyl-6-cyclopentyl-phenol, o- und m-Chlorphenol, 2,3,6-Trimethylphenol. Bevorzugt sind Phenol, o- und m-Kresol, 2,6-Dimethylphenol, o-tert.-Butylphenol und o-Phenylphenol; besonders bevorzugt ist Phenol.

Geeignete Ketone enthalten wenigstens eine aliphatische Gruppe an der Carbonylfunktion; genannt seien beispielsweise Aceton, Methylethylketon, Methylpropylketon, Methylisopropylketon, Diethylketon, Acetophenon, Cyclohexanon, Cyclopentanon, Methyl-, Dimethyl- und Trimethylcyclohexanone, die auch geminale Methylgruppen aufweisen können wie 3,3-Dimethyl-5-methylcyclohexanon (Hydroisophoron). Bevorzugt sind Aceton, Acetophenon, Cyclohexanon und dessen Methylgruppen tragende Homologe, besonders bevorzugt ist Aceton.

Das Molverhältnis von aromatischer Hydroxyverbindung zu Keton beträgt im allgemeinen 5:1 bis 25:1, bevorzugt 7:1 bis 20:1, besonders bevorzugt 8:1 bis 18:1, bezogen auf die Gesamtreaktion.

Das eingesetzte Eduktgemisch kann geringe Mengen Wasser enthalten, bevorzugt weniger als 1, besonders bevorzugt weniger als 0,6 und ganz besonders bevorzugt weniger als 0,3 Gew.-%.

Die als Katalysatoren eingesetzten Ionentauscherharze und die als Cokatalysatoren verwendeten Mercaptoverbindungen sind dem Fachmann bekannt (US-PS 2 468 982, 2 623 908; 2 775 620; DE-OS 36 19 450; 37 27 641).

Die Anzahl der Reaktoren beträgt mindestens zwei und wird aus Gründen der Wirtschaftlichkeit in der Regel nicht mehr als acht, bevorzugt nicht mehr als sechs, besonders bevorzugt nicht mehr als vier betragen.

Die Aufteilung der Ketonmenge auf die Reaktoren erfolgt so, daß die Menge pro Reaktor abnimmt mit steigender Temperatur im Reaktor. Bei zwei Reaktoren wird auf den ersten Reaktor 60 bis 90 %, vorzugsweise 65 bis 85 %, bei drei Reaktoren 40 bis 80 %, vorzugsweise 50 bis 70 % auf den ersten Reaktor und 10 bis 40 %, vorzugsweise 15 bis 35 % auf den zweiten Reaktor und der jeweilige Rest auf den dritten Reaktor gegeben.

Die Belastung, definiert als Menge (in kg) Eduktgernisch pro Liter Katalysator im Betriebszustand (gequollen) und pro Stunde liegt pro Reaktor bei 0,2 bis 2,0, bevorzugt 0,3 bis 1,7, besonders bevorzugt 0,4 bis 1,5 kg/l·h. Die Belastung sollte in der Regel so gewählt werden, daß der Umsatz an Aceton nach dem letzten Reaktor mindestens 75 %, besser ≥ 80 %, bevorzugt ≥ 85 % beträgt.

Es ist nicht erforderlich, alle Reaktoren mit gleicher Belastung zu betreiben. Vielmehr kann es für eine weitere Erhöhung der Selektivität vorteilhaft sein, die Belastung der Reaktoren in Richtung steigenden Umsatzes von Reaktor zu Reaktor zu erhöhen. Beispielsweise kann in einer Anlage mit drei Reaktoren der erste Reaktor mit 0,3, der zweite Reaktor mit 0,6 und der dritte Reaktor mit 0,8 kg/l·h betrieben werden.

Für eine wirksame Verminderung der Beiproduktmenge ist es sehr wichtig, daß vor dem Eintritt in das jeweilige Katalysatorbett das Keton völlig homogen im Reaktionsgemisch verteilt wird, was durch den Einsatz von Düsen, statischen Mischern, Rührbehältern, Kreiselpumpen oder anderen dem Fachmann geläufigen Mischapparaturen erreicht werden kann.

Die in Reihe geschalteten Reaktoren werden in Richtung fortschreitender Umsetzung mit steigenden Temperaturen betrieben. Zwischen Anfang und Ende der Reaktorkaskade wird ein ansteigendes Temperaturprofil im Temperaturbereich von 35 bis 85°C, vorzugsweise 38 bis 75°C, besonders bevorzugt 40 bis 70°C, ganz besonders bevorzugt 42 bis 68°C eingestellt. Die Temperaturunterschiede von einem zum nächsten Reaktor werden in der Regel umso geringer sein, je mehr Reaktoren durchlaufen werden sollen. Es ist auch möglich, zwei aufeinanderfolgende Reaktoren mit gleicher Temperatur zu betreiben.

Da in Festbettreaktoren keine Durchmischung stattfindet und die Abführung der Reaktionswärme aus dem Reaktionsgemisch schwierig ist, werden solche Reaktoren in der Regel adiabatisch betrieben, was zur Erwärmung des Reaktionsgemischs führt. Es ist daher meist zweckmäßig, das Reaktionsgemisch zwischen den einzelnen Reaktoren zu kühlen, wobei darauf zu achten ist, daß ein Auskristallisieren von Bis(4-hydroxyaryl)alkan, das ein Verstopfen der Rohrleitungen zur Folge hätte, vermieden wird.

### Beispiele

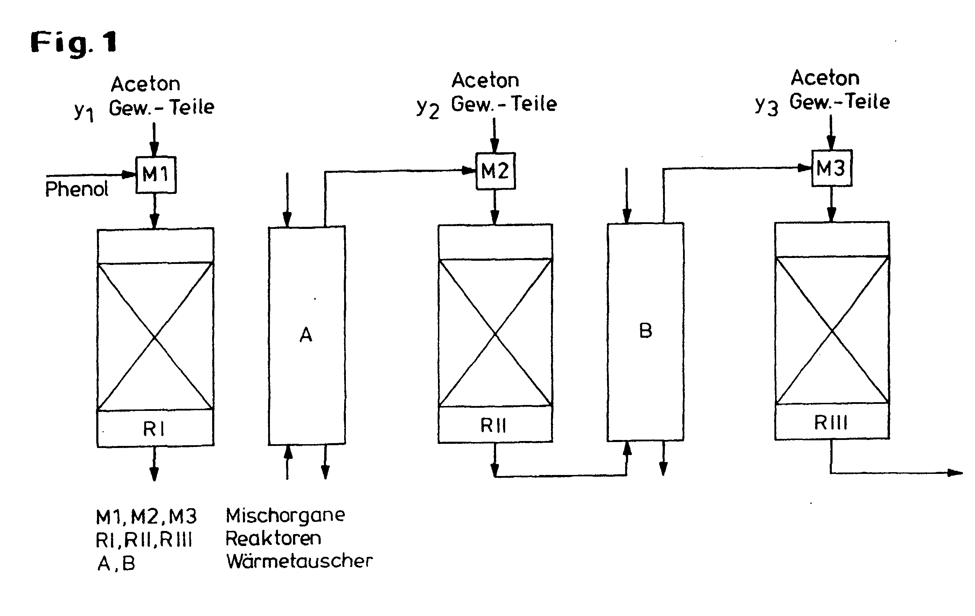

Der Versuchsaufbau ist in Figur 1 schematisch wiedergegeben. In den ersten einer Serie von 3 hintereinander geschalteten, mit einem mit 2 Gew.-% Divinylbenzol vernetzten und mit 3,5 Gew.-% Cysteamin beladenen sulfonierten Styrolharz gefüllten Festbettreaktoren wurde eine Mischung aus 100-Y₁-0,2 Gew.-% Phenol, Y₁ Gew.-% Aceton und 0,2 Gew.-% Wasser mit einer Temperatur je nach Beispiel von 45 bis 56°C und einer Belastung von 0,2 bis 0,8 kg/ 1·h unter N₂ durch das Katalysatorbett geschickt.

Das den Reaktor verlassende Produktgemisch wurde mit Y₂ Gew.-Teilen Aceton vermischt und je nach Beispiel bei 51 bis 68°C und einer Belastung von 0,2 bis 0,8 kg/ l·h durch das zweite Katalysatorbett geleitet.

Dem aus dem 2. Reaktor abfließenden Produktgemisch wurden Y₃ Gew.-Teile Aceton zugemischt und bei 51 bis 76°C und 0,2 bis 0,8 kg/ l·h durch den 3. Reaktor geleitet.

Der Umsatz an Aceton betrug U %. Jedes Beispiel wurde einige 100 Stunden lang durchgeführt. Die durch tägliche Analysen erhaltene gemittelte Zusammensetzung der Reaktionsgemische nach dem 3. Reaktor zeigt die Tabelle 1

**Tabelle 1**

| Nr. | Belastung | Acetonaufteilung Gew.-% | | | Temperatur °C | | | Acetonumsatz | Bisphenol A | o,o'-Bisphenol | o,p'-Bisphenol | Chromane | Indane |
|---|---|---|---|---|---|---|---|---|---|---|---|---|---|
| | | Y₁ | Y₂ | Y₃ | R.I | R.II | R.III | U % | % | % | % | % | % |
| 1 | 0,2 | 2,0 | 1,3 | 0,6 | 54 | 64 | 74 | 100 | 93,34 | 0,09 | 5,75 | 0,14 | 0,10 |
| a* | 0,2 | 1,3 | 1,3 | 1,3 | 54 | 64 | 74 | 99,8 | 92,83 | 0,10 | 6,10 | 0,27 | 0,22 |
| b* | 0,2 | 3,9 | 0 | 0 | 74 - | | - | 100 | 91,70 | 0,14 | 6,65 | 0,53 | 0,25 |
| 2 | 0,6 | 2,0 | 1,3 | 0,6 | 56 | 68 | 76 | 99,8 | 94,47 | 0,07 | 4,89 | 0,09 | 0,03 |
| c* | 0,6 | 1,3 | 1,3 | 1,3 | 54 | 64 | 74 | 98,8 | 93,16 | 0,10 | 5,87 | 0,21 | 0,18 |
| 3 | 0,8 | 2,0 | 1,3 | 0,6 | 56 | 68 | 76 | 94,2 | 95,18 | 0,07 | 4,13 | 0,08 | 0,07 |
| 4 | 0,6 | 2,0 | 1,3 | 0,6 | 50 | 59 | 63 | 98,1 | 96,10 | 0,06 | 3,27 | 0,08 | 0,02 |
| 5 | 0,6 | 2,0 | 1,3 | 0,6 | 45 | 51 | 57 | 95,3 | 96,95 | 0,04 | 2,65 | 0,06 | 0,02 |

| | | | | | | | | | | | | | |
|---|---|---|---|---|---|---|---|---|---|---|---|---|---|
| * Vergleichsbeispiel | | | | | | | | | | | | | |

Ohne Acetonaufteilung (Vergl. b) wird die niedrigste Selektivität für Bisphenol A (BPA) erzielt. Arbeitet man mit der gleichen Acetonmenge, die aber auf 3 Reaktoren (R.I, RII, R.III) zu gleichen Teilen (Vergl. a) aufgeteilt wird bei gleichzeitigem Anlegen eines Temperaturgradienten an die Reaktorkette, so steigt die BPA-Selektivität. Verteilt man schließlich erfindungsjemäß die Acetonmenge so auf die Reaktoren, daß der Anteil pro Reaktor mit steigender Temperatur sinkt, so ist unter sonst gleichen Bedingungen eine weitere Selektivitätserhöhung zu beobachten. Wird die Belastung des Katalysators erhöht, findet man eine weiter verbesserte BPA-Selektivität. Bei Verkleinerung des Temperaturgradienten lassen sich noch einmal bessere Selektivitäten erzielen.

Daraus ergibt sich, daß die Selektivität für BPA steigt mit einer ungleichmäßigen Acetonaufteilung, bei der der Reaktor I den höchsten Anteil, Reaktor II einen mittleren und Reaktor III mit der höchsten Temperatur den kleinsten Anteil erhält, die Temperatur und der Temperaturgradient möglichst niedrig und die Belastung der Reaktoren möglichst hoch gehalten werden.

Dagegen wird laut US-PS 4 400 555 bei einem Prozeß mit zwei bei gleicher Temperatur betriebenen Reaktoren eine höhere Selektivität für BPA erzielt, wenn der größere Teil der Acetonmenge in den zweiten Reaktor dosiert wird.

## Patentansprüche

1. Verfahren zur Herstellung von Bis-(4-hydroxyaryl)-alkanen durch heterogen katalysierte Umsetzung von aromatischen Hydroxyverbindungen mit Ketonen in mindestens zwei in Reihe geschalteten Reaktoren, die in Richtung fortschreitender Umsetzung bei steigenden Temperaturen betrieben werden, wobei die Gesamtmenge an Keton auf die einzelnen Reaktoren so aufgeteilt wird, daß der Anteil pro Reaktor umso kleiner ist, je höher die Temperatur des jeweiligen Reaktors ist.

2. Verfahren gemäß Anspruch 1, bei dem die Belastung der Reaktoren 0,2 bis 2,0 kg/l·h beträgt.

3. Verfahren gemäß Anspruch 1, bei dem die Reaktortemperaturen 35 bis 85°C betragen.

## Claims

1. Process for the production of bis-(4-hydroxyaryl)alkanes by a heterogeneously catalysed reaction of aromatic hydroxy compounds with ketones in at least two reactors connected in series, which, as the reaction progresses, are operated at higher temperatures, wherein the total quantity of ketone is divided between the individual reactors in such a manner that the higher the temperature is of the particular reactor, the smaller is the proportion per reactor.

2. Process according to claim 1, in which the loading of the reactors is 0.2 to 2.0 kg/l·h.

3. Process according to claim 1, in which the reactor temperatures are 35 to 85°C.

## Revendications

1. Procédé de préparation de bis(4-hydroxyaryl)alcanes par réaction sous catalyse hétérogène de composés hydroxylés aromatiques avec des cétones dans au moins deux réacteurs placés en série, qui fonctionnent avec la température croissant dans le sens de l'avancement de la réaction, où la quantité totale de cétone est répartie entre les différents réacteurs, de sorte que la quantité par réacteur soit plus faible lorsque la température du réacteur est plus élevée.

2. Procédé suivant la revendication 1, dans lequel la charge des réacteurs se situe dans l'intervalle allant de 0,2 à 2,0 kg/litre.heure.

3. Procédé suivant la revendication 1, dans lequel la température des réacteurs se situe dans l'intervalle allant de 35 à 85°C.
